# FASCICULE DE BREVET EUROPEEN

(11) **EP 1 562 501 B1**
(45) Date de publication et mention de la délivrance du brevet: **24.05.2006**
(21) Numéro de dépôt: 03761667.9
(22) Date de dépôt: 30.06.2003
(51) Int. Cl.: A61B 17/74

(54) **DISPOSITIF D OSTEOSYNTHESE MINI-INVASIF, NOTAMMENT POUR FRACTURES METAPHYSAIRES**
MINI-INVASIVE OSTEOSYNTHESEVORRICHTUNG,INSBESONDERE FÜR METAPHYSENFRAKTUREN
MINI-INVASIVE OSTEOSYNTHESIS DEVICE, IN PARTICULAR FOR METAPHYSEAL FRACTURES

(30) Priorité: 28.06.2002 FR 0208123
(43) Date de publication de la demande: 17.08.2005
(73) Titulaire: SARL GROUPE LEPINE, 69394 Lyon cedex 03 (FR); Langlais, Frantz, F-35000 Rennes (FR); Burdin, Philippe, 37320 Saint-Branchs (FR)
(72) Inventeur: LANGLAIS, Frantz, F-35000 Rennes (FR); BURDIN, Philippe, F-37320 Saint-Branchs (FR); LE MOALLIC, Serge, F-64300 Lanneplaa (FR); GENTIL, Pascal, F-64300 Orthez (FR)
(74) Mandataire: Jeannet, Olivier
(86) Numéro de dépôt international: PCT/FR2003/002019
(87) Numéro de publication internationale: WO 2004/002343

(56) Documents cités:
- FR-A- 2 686 788
- US-A- 3 374 786
- US-A- 5 514 138
- US-A1- 2001 049 528
- PATENT ABSTRACTS OF JAPAN vol. 015, no. 388 (C-0872), 2 octobre 1991 (1991-10-02) & JP 03 158150 A (KIJURO HAYANO), 8 juillet 1991 (1991-07-08)

## Description

La présente invention concerne les systèmes d'ostéosynthèse à plaque pour fractures métaphysaires, notamment du fémur proximal ou distal, du tibia proximal ou encore de l'humérus proximal ou distal.

La plupart des plaques d'ostéosynthèse pour les régions de la hanche et du genou comprennent un canon et une plaque diaphysaire, une vis céphalique dite de traction apte à être reçue à coulissement dans le canon, ainsi qu'une vis dite de coaptation permettant de déplacer la vis céphalique dans le canon lors de la pose de manière à ajuster la distance entre le grand trochanter et la tête fémorale pour assurer un contact osseux entre les différents morceaux d'os suite à un traumatisme de type fracture du col du fémur.

Pour réduire la taille de la voie d'abord permettant la pose de telles plaques d'ostéosynthèse, le canon et la plaque diaphysaire sont dissociés l'une de l'autre de manière à partir de deux éléments de faible encombrement, en compatibilité avec une voie d'abord mini-invasive. Le document EP-A-0 617 927, sur lequel est basé le préambule de la revendication 1, décrit une telle plaque d'ostéosynthèse. Le canon est mis en place sur la plaque diaphysaire par vissage. Cette étape est relativement longue à réaliser en per-opératoire d'autant que la plaque d'ostéosynthèse nécessite la mise en place de deux canons de cette manière. Ceci allonge d'autant le temps opératoire, ce qui est préjudiciable pour le patient.

Un but de l'invention est de fournir une plaque d'ostéosynthèse notamment diaphysaire présentant un assemblage entre le canon et la plaque qui soit simple et rapide à mettre en oeuvre.

A cet effet, on prévoit, selon l'invention, un dispositif d'ostéosynthèse à plaque notamment diaphysaire, ladite plaque comportant au moins un orifice traversant, et le dispositif comprenant en outre au moins un canon cylindrique apte à être reçu dans l'orifice traversant, et des moyens d'assemblage et de verrouillage du canon et de la plaque, caractérisé en ce que les moyens d'assemblage et de verrouillage sont agencés de sorte que l'assemblage et le verrouillage du canon et de la plaque sont réalisés lors d'un mouvement de rotation selon un axe principal du canon présentant une amplitude inférieure à 360°.

Avantageusement mais facultativement, le dispositif d'ostéosynthèse présente au moins l'une des caractéristiques supplémentaires suivantes :
- les moyens d'assemblage et de verrouillage comprennent au moins une excroissance s'étendant en saillie d'une paroi externe latérale du canon de manière radiale sur toute ou partie d'une circonférence de ladite paroi externe latérale,
- l'excroissance s'étend dans un plan transversal perpendiculaire à l'axe principal du canon,
- l'excroissance est venue de matière avec le canon,
- l'excroissance est un excentrique,
- l'excroissance est un plot,
- les moyens d'assemblage et de verrouillage comprennent au moins une gorge située dans une paroi interne de l'orifice traversant sur toute ou partie d'une circonférence de ladite paroi interne, la gorge étant apte à coopérer avec l'excroissance,
- la gorge s'étend en partie dans un plan transversal perpendiculaire à un axe principal de l'orifice,
- le dispositif comprend en outre une vis osseuse (300) apte à être reçue à coulissement dans le canon.
- la vis osseuse et le canon comprennent au moins une partie plane externe et au moins une partie plane interne respectivement aptes à coopérer l'une avec l'autre de façon à limiter une rotation de la vis osseuse dans le canon selon l'axe principal du canon.
- la vis osseuse comprend une partie tige apte à être assemblée avec une partie noyau par un simple mouvement de translation.

Un procédé de pose d'un dispositif d'ostéosynthèse tel que défini ci-dessus présente les étapes suivantes :
- réalisation à partir de l'épiphyse externe du fémur d'un trou borgne jusqu'aux environs du centre de la tête fémorale,
- mise en place dans le trou borgne de la vis osseuse,
- introduction dans l'orifice traversant de la plaque du canon et mise en place éventuelle de la plaque sur un porte-plaque,
- mise en place de la plaque sur l'épiphyse externe du fémur tout en introduisant dans le trou borgne le canon de manière à l'enfiler sur la vis osseuse,
- assemblage et verrouillage du canon sur la plaque par un mouvement de rotation dont l'amplitude est inférieure à environ 360° de manière à enclencher les moyens d'assemblage et de verrouillage,
- retrait éventuel du porte-plaque.

D'autres caractéristiques et avantages de l'invention apparaîtront lors de la description ci-après d'un mode préféré de réalisation. Sur les dessins annexés:
- la figure 1 est une vue de côté d'un dispositif d'ostéosynthèse selon l'invention, avec la vis de coaptation non installée,
- la figure 2 est une vue partielle en coupe selon la ligne C-C de la figure 1,
- les figures 3a, 3b et 3c sont respectivement une vue de côté, une vue de face et une vue en coupe selon la ligne A-A de la figure 3b d'une plaque du dispositif d'ostéosynthèse de la figure 1,
- la figure 4 est une vue en coupe selon la ligne G-G de la figure 3a,
- la figure 5 est une vue de côté d'un canon du dispositif d'ostéosynthèse de la figure 1,
- la figure 6 est une vue de face du canon de la figure 5,
- la figure 7 est une vue en coupe selon la ligne L-L de la figure 5,
- la figure 8a est une vue de côté d'une vis osseuse du dispositif d'ostéosynthèse de la figure 1,
- les figures 8b et 8c sont des vues en perspective de deux variantes de la vis osseuse de la figure 8a,
- la figure 9 est une vue en coupe selon la ligne F-F de la figure 8a,
- la figure 10 est une vue de côté d'une vis de coaptation du dispositif d'ostéosynthèse de la figure 1,
- la figure 11 est une vue en oblique du dispositif d'ostéosynthèse de la figure 1, le canon étant en position déverrouillée,
- la figure 12 est une vue en oblique du dispositif d'ostéosynthèse de la figure 1, le canon étant en position verrouillée.

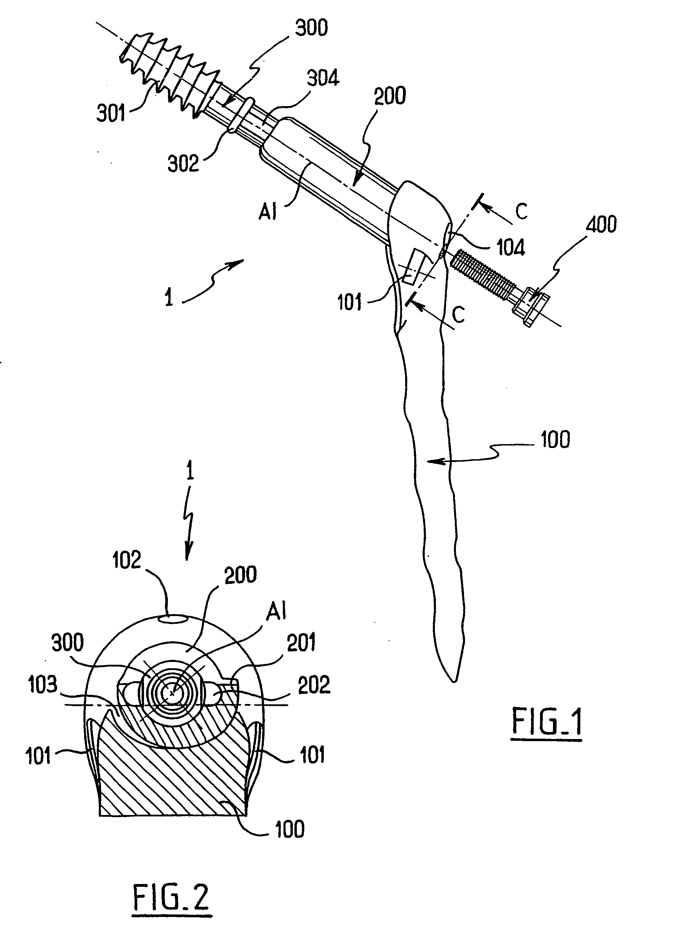

En référence tout d'abord à la figure 1, un dispositif d'ostéosynthèse 1 selon l'invention comprend ici une plaque d'ostéosynthèse diaphysaire, notamment fémorale destinée à la hanche, appelée aussi vis-plaque à compression. Le dispositif d'ostéosynthèse 1 comporte la plaque 100, un canon 200 apte à être assemblé avec la plaque 100, une vis osseuse 300, ici une vis céphalique, apte à être reçue au moins à coulissement dans le canon 200, ainsi qu'une vis dite de coaptation 400 apte à régler la position de la vis osseuse 300 au sein du canon 200 comme on le détaillera dans la suite.

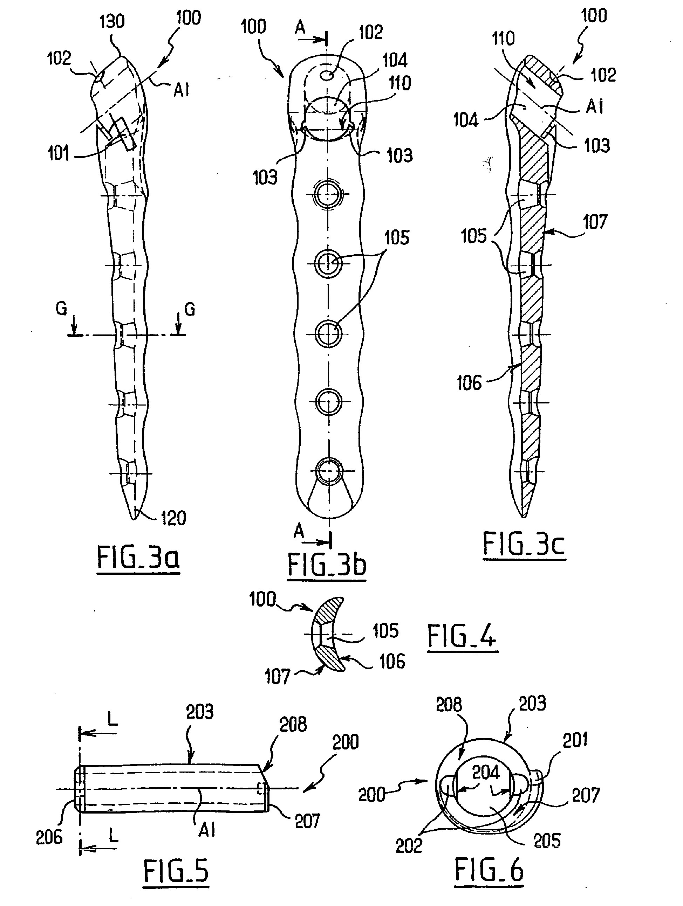

En référence aux figures 3a, 3b, 3c et 4, on va décrire plus en détail la plaque diaphysaire 100. Cette plaque 100 est de forme allongée, avec une épaisseur est inférieure à sa largeur tout en variant de préférence d'une épaisseur minimale au niveau d'une extrémité distale 120 de la plaque 100 à une épaisseur maximale au niveau d'une extrémité proximale 130 de la plaque 100. Il est à noter que de préférence l'extrémité distale 120 se termine en pointe ou en biseau ce qui permet de faciliter son insertion lors de la pose comme on le verra plus loin. Sur une grande partie d'une longueur de la plaque 100, celle-ci comporte une série d'orifices 105 traversant toute l'épaisseur de la plaque et dont les axes principaux sont perpendiculaires à un plan frontal de la plaque 100. La série d'orifices 105 est constituée ici de cinq orifices uniformément répartis de préférence sur la majeure partie de la longueur de la plaque diaphysaire 100 depuis son extrémité distale 120 jusqu'à en dessous environ de son extrémité proximale 130. Au niveau de l'extrémité proximale 130, la plaque diaphysaire 100 comporte un orifice traversant 104 de préférence cylindrique de révolution d'axe A1. L'axe A1 présente un angle avec l'axe principal de la plaque appelée ici angle cervico-diaphysaire, angle sensiblement égal à l'angle que forme naturellement un axe du col du fémur avec un axe du canal médullaire du fémur sur lequel le dispositif d'ostéosynthèse 1 est destiné à être à mis en place.

La plaque diaphysaire 100 présente une face interne 106 et une face externe 107. La face externe 107 est essentiellement concave alors que la phase interne 106 est essentiellement convexe. Il est à noter que, de préférence, les deux faces 106 et 107 ont essentiellement une forme cylindrique de révolution dont l'axe est parallèle à l'axe principal de la plaque. De plus, le rayon de courbure du cylindre de révolution formant la face interne 106 est supérieur au rayon de courbure du cylindre de révolution formant la face 107. Cette forme de la face interne 106 permet avantageusement, lors de la pose de la plaque sur le fémur, que la face 106 s'applique de manière optimale sur l'épiphyse externe dudit fémur. Pour cela, le rayon de courbure de la face 106 est sensiblement égal à celui de l'épiphyse externe du fémur sur laquelle la plaque est destinée à être posée. D'autre part, la forme particulière de la face externe 107 permet à la plaque de ne pas présenter d'angle vif saillant susceptible de blesser les tissus avoisinant l'épiphyse externe du fémur.

L'orifice traversant 104 présente sur une paroi interne 110 une gorge 103. De préférence cette gorge est située au voisinage de l'extrémité distale dudit orifice 104, extrémité située du côté de la face externe 107 de la plaque 100. De préférence, la gorge 103 est située dans un plan sensiblement perpendiculaire à l'axe A1 de l'orifice traversant 104. De préférence encore, la gorge 103 débouche vers l'extérieur au niveau de l'intersection entre la face interne 110 de l'orifice traversant 104 et la face externe 107 de la plaque 100. Dans le cas illustré aux figures 3b et 3C, la gorge 103 est située dans une partie inférieure de l'orifice 104 et débouche à l'extérieur de part et d'autre de l'axe A1.

Enfin, la plaque diaphysaire 100 présente des moyens 101, 102 permettant de mettre en place la plaque diaphysaire 100 sur un dispositif appelé porte-plaque, non représenté, servant à la pose de la plaque sur le fémur lors d'une intervention chirurgicale. Dans le cas présent, ces moyens de fixation sont composés d'une empreinte sensiblement sphérique 102 située sur l'extrémité proximale de la plaque diaphysaire 100 ainsi que de deux empreintes sensiblement cylindriques 101 situées sur les côtés de la plaque diaphysaire 100 de part et d'autre et légèrement en dessous de l'orifice traversant 104.

En référence aux figures 5 à 7, on va décrire maintenant plus en détail le canon 200 du dispositif d'ostéosynthèse 1. Le canon 200 est de forme essentiellement tubulaire cylindrique. Le canon 200 présente une face externe 203 qui est de préférence de forme cylindrique de révolution d'axe A1. De préférence, la face externe 203 présente une section constante tout le long du canon 200. D'autre part, le canon 200 comprend un orifice coaxial traversant 205 de forme essentiellement et préférentiellement cylindrique de révolution. Le canon 200 présente une extrémité proximale 206 au niveau de laquelle l'orifice 205 comporte une série de méplats 204, ici au nombre de deux et diamétralement opposés l'un à l'autre par rapport à l'axe A1 du canon et parallèles entre eux.

Le canon 200 présente, d'autre part, une extrémité distale 207 présentant une moitié inférieure plane située dans un plan sensiblement perpendiculaire à l'axe A1 et une moitié supérieure 208 plane située dans un plan présentant un angle avec l'axe de révolution A1. De manière préférentielle, cet angle est complémentaire de l'angle cervico-diaphysaire de l'axe A1 de l'orifice 104 traversant de la plaque 100, dans lequel le canon 203 est apte à être reçu à coulissement. De plus, l'extrémité 207 présente des empreintes 202, ici au nombre de deux et disposées de manière diamétralement opposées l'une par rapport à l'autre par rapport à l'axe A1 du canon. Ces empreintes 202 sont aptes à coopérer avec un outil permettant la rotation dudit canon lors de la pose de celui-ci, comme on va le voir plus loin.

Enfin, au niveau de l'extrémité distale 207 le canon 200 présente une excroissance 201 s'étendant en saillie de manière radiale de la face externe 203. Dans le cas présent, l'excroissance 203 est un excentrique situé dans un plan transversal perpendiculaire à l'axe A1 du canon 200. De manière préférentielle, cet excentrique s'étend sur un quart environ de la circonférence du canon 200. Toujours de manière préférentielle, l'excentrique 201 s'étend dans le plan de la face d'extrémité 207.

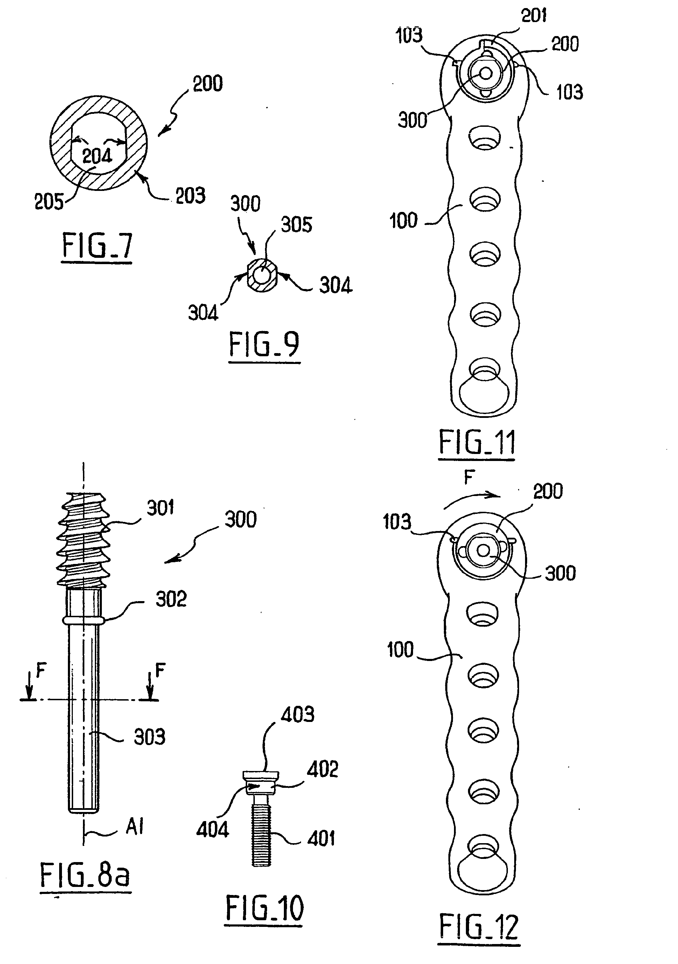

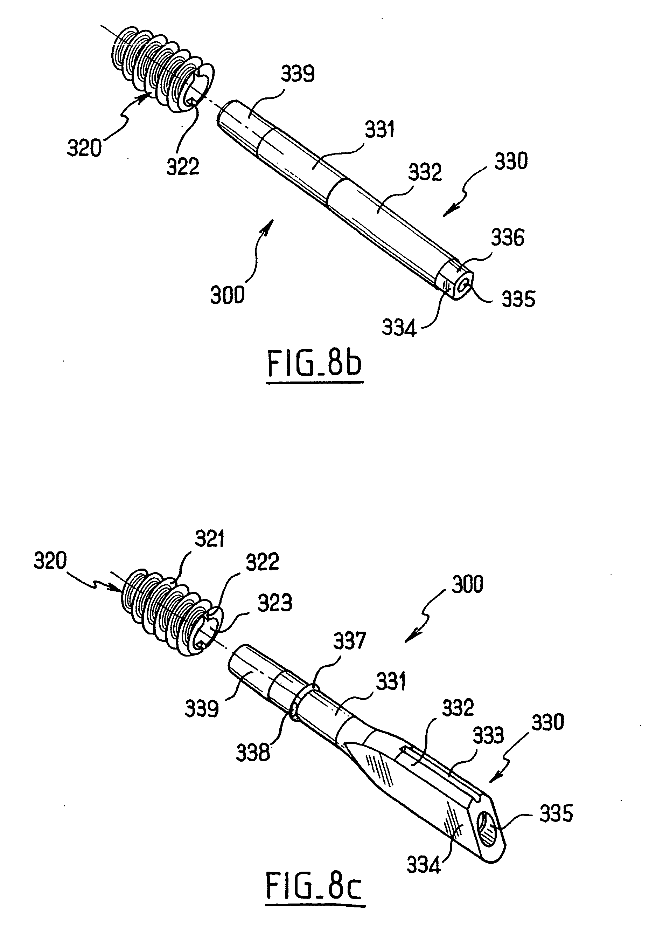

En référence aux figures 8a et 9, on va décrire la vis osseuse 300 du dispositif d'ostéosynthèse 1. La vis osseuse 300 est essentiellement de forme cylindrique de révolution d'axe A1. Elle comporte une extrémité proximale présentant des moyens d'ancrage 301 dans l'os de la tête fémorale. De préférence, les moyens d'ancrage 301 sont un filetage osseux connu en soi.

La vis osseuse 300 présente une partie distale 303 séparée de la partie proximale 301 par des moyens formant butée 302. Ces moyens formant butée 302 sont ici un bossage s'étendant en saillie de manière radiale d'une surface externe latérale de la vis osseuse 300. Le bossage 302 s'étendant de préférence sur toute la circonférence de la vis osseuse 300 et ce dans un plan perpendiculaire sensiblement à l'axe A1. La partie distale 303 comporte de part et d'autre de l'axe A1 de manière diamétralement opposée deux méplats 304 parallèles entre eux. Enfin, la vis 300 présente un perçage 305 partant de son extrémité distale jusqu'à son extrémité proximale. De préférence, ce perçage 305 est cylindrique de révolution d'axe A1. De plus, le perçage 305 présente un filetage de préférence métrique connu en soi au moins présent sur la partie distale de la vis 300.

La figure 8b illustre une vis osseuse 300 selon une variante de réalisation, qui comprenant une tige 330 et un organe d'ancrage 320 réalisés en deux parties. La tige 330 est apte à être assemblée avec l'organe d'ancrage 320, qui est ici un noyau essentiellement cylindrique de révolution comportant un filetage osseux externe 321. A une extrémité, le noyau présente une fente radiale 322 destinée à être utilisée lors d'une opération chirurgicale pour mettre en place le noyau 320 à l'aide d'un tournevis présentant une empreinte complémentaire. Le noyau 320 présente un orifice traversant coaxial 323 présentant dans sa partie centrale une section tronconique.

La tige 330 de la vis osseuse 300 présente une partie distale 332 et une partie proximale 331 formées d'un cylindre de révolution. La région d'extrémité de la partie proximale 31 présente une partie tronconique 339 se rétrécissant vers l'extrémité libre de la partie proximale 31. De préférence, le cône 339 est complémentaire du cône formé dans le noyau 320. De ce fait, les deux cônes sont aptes à coopérer l'un avec l'autre de manière à lier ensemble le noyau et la tige pour former la vis osseuse 300, et ceci par une simple translation axiale du noyau 320 et de la tige 330 l'un par rapport à l'autre. De manière préférentielle, ces cônes sont des cônes Morse.

Il est à noter que le canon présente un orifice 335 coaxial à l'axe principal de la tige, partant d'une extrémité distale 336 de la tige 330 et rejoignant son extrémité proximale.

Dans la région de l'extrémité distale 336 de la tige sont formés deux petits méplats 334 situés de part et d'autre d'un axe principal de la tige et parallèles l'un à l'autre.

Dans la variante illustrée sur la figure 8c, la tige 330 possède deux grands méplats 334 s'étendant sensiblement sur toute la longueur de la partie distale 332 de la tige 330. Entre les deux méplats 334, sur l'un des cotés convexes reliant les deux méplats, la partie distale 332 présente une rainure 333 parallèle à l'axe principal de la tige et s'étendant sur une majeure partie de la partie distale 332. La partie distale 332 présente en outre une extrémité 336 présentant une face plane préférentiellement non perpendiculaire à l'axe principal de la tige.

Bien entendu, le canon 200 est conformé en fonction des formes de la partie distale de la vis céphalique utilisée.

En référence à la figure 10, on va décrire la vis de coaptation 400. Cette vis est essentiellement de forme cylindrique de révolution. Elle présente une tête distale 402 présentant de moyens de mise en oeuvre 403 situés sur la face d'extrémité. La tête 402 présente une face 404 latérale dont le diamètre est sensiblement équivalent au diamètre de l'orifice traversant 205 du canon 200 de manière à pouvoir coopérer avec celui-ci lors de la pose. Enfin, la vis de traction 400 présente un filetage 401 en partie proximale. Le filetage 401 est apte à coopérer avec le filetage de l'orifice 305 de la vis osseuse 300.

On notera ici que la vis de coaptation 400 peut être remplacée par un ancillaire spécifique permettant

En référence aux figures 2, 11 et 12, on va décrire l'assemblage et le verrouillage du canon 200 avec la plaque 100. Dans un premier temps, le canon est inséré à coulissement dans l'orifice traversant 104 de la plaque 100 jusqu'à ce que le plan contenant l'excentrique 201 se retrouve confondu avec le plan contenant la gorge 103 de l'orifice traversant 104. A ce moment, le canon et la plaque se trouvent en position déverrouillée telle qu'illustrée à la figure 11. Ensuite, à l'aide d'un outil non représenté et présentant des moyens aptes à coopérer avec les empreintes 202 du canon 200 de manière à le mettre en oeuvre, on fait effectuer au canon 200 une rotation d'environ un quart de tour (90°) dans le sens de la flèche F. Ce mouvement de rotation introduit l'excentrique 201 dans la gorge 103, mettant le canon en position verrouillée tel qu'illustré à la figure 12 ainsi qu'à la figure 2. Sur la figure 2, il est à noter que le verrouillage s'effectue par contact entre le fond de la gorge 103 et le sommet de l'excentrique 201 qui par frottement assurent le verrouillage du canon dans la plaque.

On va maintenant décrire une technique chirurgicale permettant d'implanter un dispositif d'ostéosynthèse 1.

Dans un premier temps, un chirurgien effectue une voie d'abord dite mini-invasive c'est-à-dire de petite taille permettant d'atteindre l'épiphyse externe du fémur situé juste en dessous du grand trochanter. Il utilise une rugine spécialement conçue à cet effet. Ensuite, le chirurgien met en place contre l'épiphyse externe une plaque fantôme qui va permettre de viser l'axe du col du fémur. Pour cela, il introduit dans la plaque fantôme une broche d'environ 2,5 mm de diamètre qu'il met en place dans le fémur jusqu'à ce que la pointe de la broche atteigne la tête fémorale de manière à simuler l'axe du col du fémur. Dans une deuxième étape, le chirurgien perce un trou borgne en utilisant la broche comme axe. Ce trou borgne présente un fond situé au sein de la tête fémorale.

Ensuite, le chirurgien effectue un taraudage éventuel de ce trou pour faciliter l'insertion de la vis osseuse. Cette dernière est enfilée sur la broche puis vissée jusqu'au fond du trou borgne. Ensuite, le chirurgien installe sur le porte-plaque la plaque en utilisant les moyens de mise en oeuvre 101 et 102 de la plaque 100 après avoir inséré à coulissement le canon 200 dans l'orifice traversant 104.

Ensuite, le chirurgien retire la broche du col du fémur puis installe la plaque le long de l'épiphyse externe du fémur tout en engageant le canon dans le trou borgne.

Puis dans une étape suivante, le chirurgien enfile la partie distale de la vis osseuse dans l'orifice traversant 205 du canon de manière à ce que les deux méplats 204 du canon viennent coopérer avec les deux méplats 304 de la vis de manière à limiter le mouvement de rotation selon l'axe du canon et de la vis osseuse de cette dernière dans le canon.

Le chirurgien vient alors verrouiller le canon dans la plaque diaphysaire 100 comme indiqué précédemment, en faisant coopérer l'excentrique 201 avec la gorge 103.

Puis le chirurgien insère la vis de traction 400 dans l'orifice 205 du canon 200 puis dans l'orifice 305 de la vis osseuse en faisant coopérer le filetage de l'orifice 205 avec le filetage 401 de la vis osseuse. En fin de vissage, la surface 404 de la vis 400 vient coopérer avec l'orifice 205 du canon 400 au niveau de son extrémité distale. L'extrémité 403 de la vis 400 vient en butée contre la face d'extrémité 207 du canon 200.

Si à partir de ce moment, le chirurgien continue de visser la vis de traction, la coopération des filets 401 avec les filets de l'orifice 305 oblige la vis osseuse à effectuer un mouvement de translation de manière à ce que le repère 302 de la vis osseuse se rapproche de l'extrémité proximale 206 du canon. Ainsi, le chirurgien peut régler la compression car la vis osseuse va entraîner dans son déplacement la tête fémorale de manière à la rapprocher du grand trochanter tout en comprimant les fragments du col du fémur, dans le cas d'une fracture de ce dernier. Ceci assure une meilleure qualité de fusion des différents fragments par la suite.

Enfin le chirurgien termine la fixation de la plaque diaphysaire sur l'épiphyse externe du fémur en installant des vis osseuses à travers la série d'orifices 105, retire le porte-plaque et referme sa voie d'abord.

Bien entendu, on pourra apporter à l'invention de nombreuses modifications sans sortir du cadre de celle-ci qui est défini par les revendications. Notamment, il sera possible de remplacer l'excentrique 201 par un plot de manière à former un assemblage et verrouillage de type baïonnette. Il est possible d'utiliser une vis osseuse de type en deux parties dans lequel les moyens d'ancrage 301 sont dissociables de la tige 303.

## Revendications

1. Dispositif d'ostéosynthèse (1) à plaque (100) notamment diaphysaire, ladite plaque comportant au moins un orifice traversant (104), et le dispositif comprenant en outre au moins un canon cylindrique (200) apte à être reçu dans l'orifice traversant, et des moyens d'assemblage et de verrouillage (103, 201) du canon et de la plaque, **caractérisé en ce que** les moyens d'assemblage et de verrouillage sont agencés de sorte que l'assemblage et le verrouillage du canon et de la plaque sont réalisés lors d'un mouvement de rotation selon un axe principal (A1) du canon présentant une amplitude inférieure à 360°.

2. Dispositif selon la revendication 1, **caractérisé en ce que** les moyens d'assemblage et de verrouillage comprennent au moins une excroissance (201) s'étendant en saillie d'une paroi externe latérale (203) du canon de manière radiale sur toute ou partie d'une circonférence de ladite paroi externe latérale.

3. Dispositif selon la revendication 2, **caractérisé en ce que** l'excroissance s'étend dans un plan transversal perpendiculaire à l'axe principal du canon.

4. Dispositif selon la revendication 2 ou 3, **caractérisé en ce que** l'excroissance est venue de matière avec le canon.

5. Dispositif selon l'une des revendications 2 à 4, **caractérisé en ce que** l'excroissance est un excentrique.

6. Dispositif selon l'une des revendications 2 à 4, **caractérisé en ce que** l'excroissance est un plot.

7. Dispositif selon l'une des revendications 2 à 6, **caractérisé en ce que** les moyens d'assemblage et de verrouillage comprennent au moins une gorge (103) située dans une paroi interne (110) de l'orifice traversant sur toute ou partie d'une circonférence de ladite paroi interne, la gorge étant apte à coopérer avec l'excroissance.

8. Dispositif selon la revendication 7, **caractérisé en ce que** la gorge s'étend en partie dans un plan transversal perpendiculaire à un axe principal de l'orifice.

9. Dispositif selon l'une des revendications 1 à 8, **caractérisé en ce que** le dispositif comprend en outre une vis osseuse (300) apte à être reçue en coulissement dans le canon.

10. Dispositif selon la revendication 9, **caractérisé en ce que** la vis osseuse et le canon comprennent au moins une partie plane externe (304) et au moins une partie plane interne (204) respectivement aptes à coopérer l'une avec l'autre de façon à limiter une rotation de la vis osseuse dans le canon selon l'axe principal du canon.

11. Dispositif selon l'une des revendications 9 et 10, **caractérisé en ce que** la vis osseuse (300) comprend une partie tige (330) apte à être assemblée avec une partie noyau (320) par un simple mouvement de translation.

## Claims

1. Plate-type osteosynthesis device (1), in particular diaphyseal, having a plate (100) which includes at least one through orifice (104), and the device including moreover at least one cylindrical barrel (200) capable of being received in the through orifice, and means (103, 201) for assembling and locking the barrel and the plate, **characterised in that** the assembling and locking means are design such that the barrel and the plate are assembled and locked by a rotational movement along a main axis (A1) of the barrel with an amplitude less than 360°.

2. Device according to claim 1, **characterised in that** the assembling and locking means comprise at least one excrescency (201) protruding from a lateral external wall (203) of the barrel radially over a circumference, in all or in part, of said lateral external wall.

3. Device according to claim 2, **characterised in that** the excrescency extends in a transverse plane perpendicular to the main axis of the barrel.

4. Device according to claim 2 or 3, **characterised in that** the excrescency is formed with material taken from the barrel.

5. Device according to one of the claims 2 to 4, **characterised in that** the excrescency is an eccentric.

6. Device according to one of the claims 2 to 4, **characterised in that** the excrescency is a stud.

7. Device according to one of the claims 2 to 6, **characterised in that** the assembling and locking means comprise at least one groove (103) situated in an internal wall (110) of the through orifice over a circumference, in all or in part, of said internal wall, the groove being capable of co-operating with the excrescency.

8. Device according to claim 7, **characterised in that** the groove extends partially in a transverse plane perpendicular to a main axis of the orifice.

9. Device according to one of the claims 1 to 8, **characterised in that** the device comprises moreover a bony screw (300) capable of being received slidingly in the barrel.

10. Device according to claim 9, **characterised in that** the bony screw and the barrel comprise at least one external flat portion (304) and at least one internal flat portion (204) respectively capable of co-operating with one another in order to limit the rotation of the bony screw in the barrel along the main axis of the barrel.

11. Device according to one of the claims 9 or 10, **characterised in that** the bony screw (300) comprises a stem portion (330) capable of being assembled with a core portion (320) by a simple translational movement.

## Patentansprüche

1. Osteosynthesevorrichtung (1) mit einer insbesondere diaphysären Platte (100), wobei die Platte mindestens eine durchgehende Öffnung (104) umfasst, und die Vorrichtung außerdem mindestens eine zylindrische Hülse (200), die geeignet ist, um in der durchgehenden Öffnung aufgenommen zu werden, und Mittel zum Zusammenfügen und Verriegeln (103, 201) der Hülse und der Platte umfasst, **dadurch gekennzeichnet, dass** die Mittel zum Zusammenfügen und Verriegeln derart angeordnet sind, dass das Zusammenfügen und das Verriegeln der Hülse und der Platte bei einer Drehbewegung an einer Hauptachse (A1) der Hülse, die eine Amplitude von weniger als 360° aufweist, vorgenommen werden.

2. Vorrichtung nach Anspruch 1, **dadurch gekennzeichnet, dass** die Mittel zum Zusammenfügen und Verriegeln mindestens eine Ausstülpung (201) umfassen, der sich von einer äußeren Seitenwand (203) der Hülse vorstehend radial auf dem ganzen oder teilweisen Umfang der äußeren Seitenwand erstreckt.

3. Vorrichtung nach Anspruch 2, **dadurch gekennzeichnet, dass** die Ausstülpung sich in einer Querebene rechtwinklig zu der Hauptachse der Hülse erstreckt.

4. Vorrichtung nach Anspruch 2 oder 3, **dadurch gekennzeichnet, dass** die Ausstülpung aus dem Material mit der Hülse kommt.

5. Vorrichtung nach einem der Ansprüche 2 bis 4, **dadurch gekennzeichnet, dass** die Ausstülpung ein Exzenter ist.

6. Vorrichtung nach einem der Ansprüche 2 bis 4, **dadurch gekennzeichnet, dass** die Ausstülpung ein Stift ist.

7. Vorrichtung nach einem der Ansprüche 2 bis 6, **dadurch gekennzeichnet, dass** die Mittel zum Zusammenfügen und Verriegeln mindestens eine Auskehlung (103) umfassen, die sich in einer Innenwand (110) der durchgehenden Öffnung auf dem ganzen oder teilweisen Umfang der Innenwand befindet, wobei die Auskehlung geeignet ist, um mit der Ausstülpung zusammenzuwirken.

8. Vorrichtung nach Anspruch 7, **dadurch gekennzeichnet, dass** die Auskehlung sich zum Teil in einer Querebene rechtwinklig zu einer Hauptachse der Öffnung erstreckt.

9. Vorrichtung nach einem der Ansprüche 1 bis 8, **dadurch gekennzeichnet, dass** die Vorrichtung außerdem einen Knochennagel (300) umfasst, der dazu geeignet ist, um gleitend in der Hülse aufgenommen zu werden.

10. Vorrichtung nach Anspruch 9, **dadurch gekennzeichnet, dass** der Knochennagel und die Hülse mindestens einen ebenen äußeren Teil (304) und mindestens einen ebenen inneren Teil (204) umfassen, die jeweils geeignet sind, um miteinander zusammenzuwirken, um eine Drehung des Knochennagels in der Hülse an der Hauptachse der Hülse zu begrenzen.

11. Vorrichtung nach einem der Ansprüche 9 und 10, **dadurch gekennzeichnet, dass** der Knochennagel (300) einen Stielteil (330) umfasst, der geeignet ist, um mit einem Kernteil (320) durch eine einfache Parallelverschiebung zusammengefügt zu werden.
